(19) Europäisches Patentamt
European Patent Office
Office européen des brevets

(11) Veröffentlichungsnummer: **0 220 584**
**A2**

(12) **EUROPÄISCHE PATENTANMELDUNG**

(21) Anmeldenummer: **86114124.0**

(51) Int. Cl.⁴: **C 07 C 125/08**

(22) Anmeldetag: **13.10.86**

(30) Priorität: **26.10.85 DE 3538128**

(43) Veröffentlichungstag der Anmeldung: **06.05.87**
**Patentblatt 87/19**

(84) Benannte Vertragsstaaten: **AT BE CH DE FR GB IT LI NL**

(71) Anmelder: **BAYER AG, Konzernverwaltung RP**
**Patentabteilung, D-5090 Leverkusen 1 Bayerwerk (DE)**

(72) Erfinder: **Fauss, Rudolf, Dr., Gerstenkamp 10,**
**D-5000 Köln 80 (DE)**
Erfinder: **Riebel, Hans-Jochem, Dr., In der Beek 92,**
**D-5600 Wuppertal 1 (DE)**

(54) **Verfahren zur Herstellung von Aryl-cyanamiden.**

(57) Die Erfindung betrifft ein Verfahren zur Herstellung von Aryl-cyanamiden durch Umsetzung von Arylaminen der allgemeinen Formel (I)

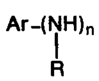
(I)

worin
Ar für gegebenenfalls zusätzlich substituiertes Aryl steht,
R für Wasserstoff oder Alkyl steht und
n die Zahlen 1, 2 oder 3 bedeutet,

(wovon jedoch 2-Nitro- und 4-Nitroanilin sowie solche Arylamine, die eine gleich geringe oder eine noch geringere Nucleophilie als 2- bzw. 4-Nitroanilin besitzen, ausgenommen sind),

mit Chlorcyan (C1-CN), welches erstmals die Herstellung auch von Cyanamiden schwach-basischer Arylamine auf diesem Wege in hohen Ausbeuten und hoher Reinheit gestattet.

Das Verfahren ist dadurch gekennzeichnet, daß man die Reaktion zu Beginn in homogener flüssiger Phase durchführt unter Verwendung von Essigsäure (welche gegebenenfalls durch Wasser und/oder durch ein mit Wasser mischbares organisches Hilfslösungsmittel verdünnt ist) als Reaktionsmedium, und daß man auf jede Aminogruppe pro Mol Arylamin (I) 1-2 Mol Chlorcyan und 1-1,5 Moläquivalente einer Hilfsbase einsetzt, wobei zu jedem Zeitpunkt der Reaktion dem Reaktionsgemisch mehr Chlorcyan als Hilfsbasenäquivalente zugesetzt werden, so daß der pH-Wert des Reaktionsgemisches unterhalb von 7 bleibt.

Die so herstellbaren Aryl-Cyanamide besitzen die Formel (II)

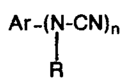
(II)

Worin Ar, R und n die vorstehenden Bedeutungen haben, und können als Zwischenprodukte zur Herstellung z.B. von entzündungshemmenden Verbindungen, Coccidiostatica, Sedativa, Analgetica, Narkotica und Diuretica verwendet werden. Die Polycyanamide der Formel (II), mit n = 2 oder 3, können ferner in Polymere mit wertvollen Eigenschaften überführt werden.

EP 0 220 584 A2

BAYER AKTIENGESELLSCHAFT 5090 Leverkusen, Bayerwerk
Konzernverwaltung RP
Patentabteilung Bi/em-c
IVa/ZP

# Verfahren zur Herstellung von Aryl-cyanamiden

Die Erfindung betrifft ein neues, breit anwendbares Verfahren zur Herstellung von Aryl-cyanamiden durch Umsetzung der entsprechenden Arylamine mit Chlorcyan, welches es erstmals gestattet, auch schwach-basische Arylamine in hoher Reinheit mit sehr guten Ausbeuten auf diesem Weg in die gewünschten Cyanamide zu überführen. Die aromatischen Cyanamide sind wertvolle Zwischenprodukte in verschiedenen Bereichen der organischen Chemie.

Nach Angaben der allgemeinen Übersichtsliteratur sind Amine mit Halogencyanen zu Cyanamiden umsetzbar (Ferry: Reaktionen der organischen Synthese; S. 668 (1978); Houben-Weyl: Methoden der organischen Chemie, Bd. E 4, S. 981/82 und 988/89; V. Migrdichian: The Chemistry of Organic Cyanogen Compounds, S. 102 ff. (1947)).

Bei einem genauen Studium der Originalliteratur wird jedoch schnell festgestellt, daß für die Reaktionen von Halogencyanen mit schwach nucleophilen bzw. schwach basischen aromatischen Aminen keine Beispiele mit Angaben über befriedigende Ausbeuten und Reinheiten der entsprechenden Cyanamide vorliegen; stärker nucleophile bzw. basische Amine ergeben zwar meist befriedigende bis gute Ausbeuten, jedoch sind in diesen Fällen die Produkte verunreinigt und ein aufwendiger Reinigungsschritt wird erforderlich.

Als schwach nucleophile aromatische Amine im Sinne der vorliegenden Erfindung gelten Amine mit einer geringeren Nucleophilie als unsubstituiertes Anilin. In Ermangelung einer allgemein anerkannten brauchbaren Meßgröße für die Nucleophilie wird - wie in der Literatur üblich - als Näherung die Basizität als Kriterium herangezogen.

Die systematischste Arbeit zur Umsetzung von Halogencyanen mit Aminen, welche auch die schwach-basischen aromatischen Amine im Sinne der vorliegenden Erfindung umfaßt, ist jene von M.P. Pierron (Ann. chim. phys. [8] 15, S. 145 - 181 (1908)).

Wie Pierron auf S. 157 ff. dieser Publikation schreibt, gelingt es ihm, aromatische Amine in wäßriger bzw. alkoholisch-wäßriger Suspension bzw. Lösung in Gegenwart eines Alkalibicarbonates mit Bromcyan umzusetzen. Pierron verwendet Bromcyan, da dieses - neben der einfachen Handhabbarkeit im Labor - weniger schnell als das technisch bedeutsame Chlorcyan in Gegenwart von Wasser und/oder Alkali hydrolysiert (S. 158 oben). Zudem sind aufgrund des Siedepunktes mit Bromcyan höhere Reaktionstemperaturen zu erreichen.

Nach eigenen Versuchen der Anmelderin sind aber die Ausbeuten von Pierron zu hoch gegriffen bzw. ist die Reinheit

der erhaltenen Cyanamide unzureichend. Pierron erhält beispielsweise aus 3-Nitroanilin in einer Rohausbeute von 76 % d. Th. das entsprechende 3-Nitrophenylcyanamid.

Bereits bei den in den Patentschriften US-A-3 830 928 bzw. DE-A-2 334 821 beschriebenen Arbeiten wurde dies nachgestellt. Die Autoren mußten das - in Anlehnung nach der von Pierron gegebenen Vorschrift hergestellte - Rohprodukt reinigen und erzielten dann eine Ausbeute von nur 41 % der Theorie an Reinsubstanz (3-Nitrophenylcyanamid).

Nach der Verfahrensweise von Pierron lassen sich gemäß eigenen Untersuchungen der Anmelderin schwach-basische Amine mit Chlorcyan nicht in befriedigender Ausbeute und Reinheit zu den entsprechenden Cyanamiden umsetzen.

Ebenso sind die anderen sonst üblichen Verfahren für die Herstellung von Cyanamiden (Lit.: Synthesis 1976, S. 591; J. Chem. Soc., Perkin Trans. I 1984, S. 147 ff) auf die Umsetzung schwach-basischer Amine nicht übertragbar. Nach eigener Untersuchung der Anmelderin kommt es entweder zu keiner Reaktion oder zu den bereits von Pierron beschriebenen Nebenreaktionen, z.B. Verseifung des Chlorcyans im alkalischen Medium oder Weiterreaktion der Cyanamide mit noch nicht umgesetztem Amin zu Guanidinen bzw. polymeren Folgekörpern. Allenfalls werden sehr geringe Ausbeuten erzielt.

Z. B. wird beim Nacharbeiten der Synthese von 4-Chlorphenylcyanamid nach Journal of the Indian Institute of Science, Section A, Bangalore, 29 A, S. 5 (1946) - einer Analogiereaktion zum Verfahren von Pierron - kaum Produkt erhalten, sofern mit Chlorcyan gearbeitet wird.

Itaya und Ogawa (Tetrahedron 38, 176 (1982)) beschreiben die Umsetzung von bestimmten Alkylaminoimidazolen mit Bromcyan in einem Essigsäure-Natriumacetatpuffer zu den entsprechenden Cyanamiden. Die Autoren verwenden einen großen Überschuß Bromcyan (5-molar), arbeiten während der gesamten Reaktion mit einer Suspension und erhalten Ausbeuten zwischen nur 25 und 56 % der Theorie.

Für ein technisches Verfahren ist diese Synthesevariante aber nicht geeignet. Bromcyan ist auf Grund seiner physikalischen Eigenschaften (Kp. = 62° C, Fp. = 50° C) sowie seiner Lagerinstabilität (Organic Synthesis, Coll. Vol. II, S. 151, Note 4) für technische Reaktionen ungeeignet. Zudem bringt der Einsatz solch großer überstöchiometrischer Mengen einer derart giftigen Chemikalie erhebliche Aufarbeitungs- und Sicherheitsprobleme mit sich.

Wird bei dem Verfahren von Itaya und Ogawa an Stelle von Bromcyan mit Chlorcyan gearbeitet, der Überschuß des Halogencyans beispielsweise auf die doppelt molare Menge zurückgenommen und die Umsetzung mit schwach-basischen Aminen durchgeführt, so werden auch nur unreine Cyanamide in unbefriedigender Ausbeute erhalten.

Bei der Umsetzung der stärker nucleophilen bzw. basischen aromatischen Amine tritt insbesondere die Nebenreaktion der Guanidinbildung bzw. Harnstoffbildung in den Vordergrund. Bacaloglu und Mitarbeiter (J. Praktische Chemie 317 (1975) 6, S. 907-18; J. Chem. Soc. Perk. Trans. II 1976, 5, S. 524-31) haben dies untersucht und sinnvoll erklären können.

Die DE-A-2 019 214 beschreibt ein Verfahren zur Herstellung von Biscyanamiden stark-basischer Amine. Hier werden in den Beispielen 2 - 10 verschiedene Varianten der Chlorcyan-Amin-Reaktion näher beschrieben. Die Ausbeuten an Rohprodukt sind zwar durchweg befriedigend bis gut, jedoch beträgt der Cyanamidgehalt der Rohprodukte nur bis zu 90 %. Das Einsatzgebiet der Biscyanamide liegt hauptsächlich auf dem Polymergebiet, und hier sind Cyanamidgehalte von max. 90 %, wie beschrieben, nicht ausreichend. Um die gewünschten Cyanamide in genügend reiner Form zu erhalten, muß eine verlustreiche Reinigungsstufe durchlaufen werden; eine Ausbeuteangabe an Reinsubstanz fehlt.

Es bestand demnach ein dringendes technisches Bedürfnis nach einem allgemein anwendbarem Verfahren zur Herstellung von Cyanamiden aromatischer Amine, eingeschlossen schwachbasischer Amine, welches den Einsatz von Chlorcyan ermöglicht und bei einem möglichst geringen Überschuß desselben eine hohe Ausbeute bei hoher Reinheit an Produkt liefert.

Es wurde nun gefunden, daß man Aryl-cyanamide durch Umsetzung von Arylaminen der allgemeinen Formel I

$$Ar - (\underset{\displaystyle R}{\underset{|}{N}}H)_n \qquad (I)$$

worin

Ar für gegebenenfalls zusätzlich substituiertes Aryl steht,

R für Wasserstoff oder Alkyl steht und

n die Zahlen 1, 2 oder 3 bedeutet,

(wovon jedoch 2-Nitro- und 4-Nitroanilin sowie solche Arylamine, die eine gleich geringe oder eine noch geringere Nucleophilie als 2- bzw. 4-Nitroanilin besitzen, ausgenommen sind),

mit Chlorcyan (Cl-CN) dann in hohen Ausbeuten bei hoher Reinheit erhält, wenn man die Reaktion zu Beginn in homogener flüssiger Phase durchführt unter Verwendung von Essigsäure (welche gegebenenfalls durch Wasser und/oder durch ein mit Wasser mischbares organisches Hilfs-Lösungsmittel verdünnt ist) als Reaktionsmedium, und wenn man auf jede Aminogruppe pro Mol Arylamin (I) 1 - 2 Mol Chlorcyan und 1 - 1,5 Moläquivalente einer Hilfsbase einsetzt, wobei zu jedem Zeitpunkt der Reaktion dem Reaktionsgemisch mehr Chlorcyan als Hilfsbasenäquivalente zugesetzt werden, so daß der pH-Wert des Reaktionsgemisches unterhalb von 7 bleibt.

Die nach dem erfindungsgemäßen Verfahren herstellbaren Aryl-Cyanamide können durch die allgemeine Formel (II)

$$Ar-\left(\underset{\displaystyle R}{\underset{|}{N}}-CN\right)_n \qquad (II)$$

worin Ar, R und n die oben bei Formel (I) angegebenen Bedeutungen haben, beschrieben werden und umfassen damit auch die Cyanamide schwach-basischer Arylamine, ausgenommen lediglich Cyanamide der unter Formel (I) bezeichneten Arylamine mit zu geringer Nucleophilie.

Im Hinblick auf den oben dargelegten Stand der Technik ist es als sehr überraschend zu bezeichnen, daß es unter den Bedingungen des erfindungsgemäßen Verfahrens gelingt, aromatische Amine - eingeschlossen schwach-basische Arylamine - mit hohen Ausbeuten bei gleichzeitig hoher Reinheit in die entsprechenden Aryl-cyanamide zu überführen. Von besonderem Vorteil ist dabei die breite Anwendbarkeit des neuen Verfahrens; die Reaktion versagt lediglich bei Arylaminen, welche zu schwach nucleophil sind. So läßt sich beispielsweise 3-Nitroanilin noch gut umsetzen, während 2- und 4-Nitroanilin bereits nicht mehr mit Chlorcyan reagieren.

Bei Verwendung von 3-Fluoranilin als Ausgangsstoff kann das erfindungsgemäße Verfahren durch folgendes Reaktionsschema wiedergegeben werden:

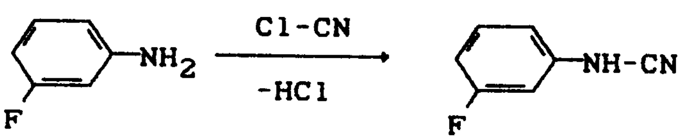


Die als Ausgangsstoffe zu verwendenden Arylamine sind durch die Formel (I) allgemein definiert. Bevorzugt sind solche Arylamine der Formel (I), worin Ar für Phenyl,

1-Naphthyl oder 2-Naphthyl steht, wobei diese Reste gegebenenfalls ein- oder mehrfach, gleich oder verschieden substituiert sein können, vorzugsweise durch folgende Substituenten: Fluor, Chlor, Brom, Iod, ($C_1$-$C_4$)-Alkyl (insbesondere Methyl und Ethyl), ($C_1$-$C_4$)-Alkoxy (insbesondere Methoxy und Ethoxy), ($C_1$-$C_4$)-Alkylthio (insbesondere Methylthio), ($C_1$-$C_2$)-Alkylsulfonyl, (insbesondere Methylsulfonyl), Trifluormethyl, Trifluormethoxy, Trifluormethylthio, Nitro, ($C_1$-$C_4$)-Alkoxycarbonyl (insbesondere Methoxy- und Ethoxycarbonyl), Aminocarbonyl, ($C_1$-$C_4$)-Alkyl- und Di-($C_1$-$C_4$)-alkylaminocarbonyl, Phenylaminocarbonyl (welches gegebenenfalls im Phenylring substituiert ist, z.B. durch Halogen, Niederalkyl oder Nitro), ($C_1$-$C_4$)-Acylamino (z.B. Acetylamino), Di-($C_1$-$C_4$)-alkylamino (z.B. Dimethylamino), Aminobenzyl, Aminophenoxy oder Aminophenylsulfonyl; und wobei die genannten Arylreste auch durch ankondensierte (vorzugsweise 5-gliedrige) heterocyclische Ringe substituiert sein können;

worin weiter R bevorzugt für Wasserstoff oder ($C_1$-$C_4$)-Alkyl (insbesondere Methyl und Ethyl) steht und

n bevorzugt für 1 und 2 steht.

Besonders bevorzugt als Ausgangsstoffe sind solche Arylamine der allgemeinen Formel (I), deren Nucleophilie (bzw. Basizität) kleiner ist als die von unsubstituiertem Anilin.

Als spezielle Beispiele für Ausgangsverbindungen, die ebenfalls von der allgemeinen Formel (I) umfaßt werden, seien folgende Arylamine genannt:

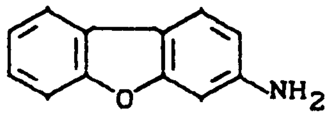, sowie 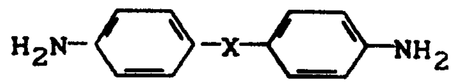,

wobei X für $-CH_2-$, $-O-$ oder $-SO_2-$ stehen kann.

Als typische Beispiele für aromatische Diamine der allgemeinen Formel (I), mit n = 2, seien folgende Verbindungen genannt:

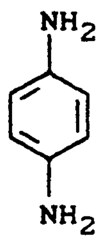, 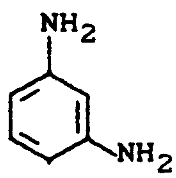, 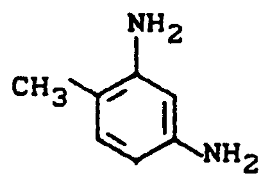, 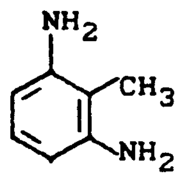.

Als Ausgangsstoffe können grundsätzlich alle aromatischen Amine (Mono-, Di- und Triamine) eingesetzt werden, die eine stärkere Nucleophilie als 2- bzw. 4-Nitroanilin besitzen und deren weitere Substituenten gegen Chlorcyan inert sind, soweit diese Amine - z.B. in Form ihrer Acetate - in dem verwendeten essigsauren Reaktionsmedium löslich sind.

Die angegebene Grenze ergibt sich aus der einfachen Tatsache, daß zu schwach nucleophile Arylamine im erfindungsgemäßen Verfahren nicht mehr mit Chlorcyan reagieren. Ob ein Arylamin noch als Ausgangsprodukt für das erfindungsgemäße Verfahren geeignet ist, läßt sich somit ohne weiteres durch einen einfachen Vorversuch bestimmen.

Selbstverständlich kann bei dem erfindungsgemäßen Verfahren anstelle von Chlorcyan auch Bromcyan eingesetzt werden, jedoch bringt dies keine Vorteile.

Zur Erzielung hoher Ausbeuten an reinen Produkten ist es erforderlich, die Umsetzung der aromatischen Amine mit Chlorcyan in der Anfangsphase in homogener flüssiger Phase durchzuführen. Wenn zu Beginn der Reaktion das Ausgangs-Amin nicht vollständig gelöst ist, wird bei manchen Aminen ein Teil der Aminmenge nicht umgesetzt und man erhält entsprechend verunreinigte Endprodukte.

Bevorzugt wird in verdünnter Essigsäure als Reaktionsmedium gearbeitet, gegebenenfalls unter Zusatz eines geeigneten Hilfslösungsmittels.

Die benötigte Menge an Essigsäure, Wasser und gegebenenfalls Hilfslösungsmittel ist abhängig von der Löslichkeit des Ausgangs-Amins bzw. seines Acetats. Bei einigen Aminen, deren Acetate gut wasserlöslich sind, wie z.B. 4-Chloranilin, genügt dazu eine sehr verdünnte Essigsäure. Bei anderen Aminen, z.B. 3,4-Dichloranilin, muß eine höher konzentrierte Essigsäure verwendet werden. Die benötigten Mengen können jeweils in einfachen Vorversuchen leicht ermittelt werden. In jedem Falle wird auf jede Aminogruppe pro Mol Arylamin (I) mindestens 1 Mol Essigsäure, im allgemeinen 1 - 50 Mol, vorzugsweise 1 -20 Mol eingesetzt.

Als Hilfslösungsmittel können wasserlösliche organische Lösungsmittel mitverwendet werden. Hierzu gehören wasserlösliche Alkohle und Ether wie z.B. Methanol, Ethanol, Propanol, Isopropanol, Glycol, Diethylenglycol, Triethylenglycol, Glycolmonomethylether, Diethylenglycolmonomethylether, Tetrahydrofuran und Dioxan; ferner können verwendet werden: Lösungsmittel wie Aceton, Ameisensäure, Dimethylformamid, N-Methylpyrrolidon, Tetramethylharnstoff oder Sulfolan (Tetramethylensulfon).

Besonders bewährt hat sich Ethanol als Hilfslösungsmittel; dadurch kann der sonst in vielen Fällen als Lösungsmittel benötigte Essigsäureüberschuß weitgehend ersetzt werden.

(Es ist auch möglich, bei Verwendung von Ethanol die restliche Menge Essigsäure durch Benzoesäure zu ersetzen; hierin wird aber kein Vorteil gesehen). Am meisten bevorzugt sind entweder ein Essigsäureüberschuß als Lösungsmittel oder billige Alkohle wie Methanol und Ethanol als Hilfslösungsmittel.

Als Hilfsbasen sind in erster Linie Alkalihydroxide, wie Natrium- und Kaliumhydroxid, bevorzugt in Form ihrer wäßrigen Lösungen zu verwenden. Aber auch die Alkalisalze von schwachen Säuren, wie beispielsweise Natriumcarbonat, Kaliumhydrogencarbonat und Kaliumacetat sind einsetzbar.

Die Reaktionstemperatur kann in einem bestimmten Bereich variiert werden; sie ist so zu wählen, daß das Reaktionsmedium flüssig-homogen bleibt, z.B. daß die - gegebenenfalls verdünnte - Essigsäure nicht auskristallisiert. Damit kommt der Temperaturbereich von -20° C bis +60° C in Betracht. Vorzugsweise arbeitet man zwischen 0° C und 40° C, besonders vorteilhaft zwischen 5 und 25° C. (Eine geringe Menge auskristallisierter Essigsäure kann aber in einigen Fällen erwünscht sein, da durch die Schmelzwärme ein Teil der Reaktionswärme abgeführt werden kann). Im allgemeinen arbeitet man bei Normaldruck.

Wird unter diesen Bedingungen der homogenen Lösung des Ausgangs-Amins Chlorcyan zugesetzt, so setzt sofort die Bildung des entsprechenden Cyanamids ein. Der pH-Wert der Lösung sinkt und muß durch Zugabe von Hilfsbase (z.B.

NaOH) im schwach-sauren Bereich (pH <7 und >3) gehalten werden, da anderenfalls die Reaktion zum Stillstand kommt oder unerwünschte, störende Nebenreaktionen eintreten.

Andererseits muß auch ein alkalisches Medium vermieden werden, weil hier ebenfalls störende Nebenreaktionen eintreten würden, welche zu verunreinigten Produkten führen. Es hat sich als besonders zweckmäßig und vorteilhaft erwiesen, daß während der Reaktion stets ein geringer Chlorcyan-Überschuß relativ zur Menge der eingesetzten Hilfsbase garantiert ist (pH < 7).

Bei der Durchführung des Verfahrens setzt man auf jede Aminogruppe pro Mol Arylamin (I) im allgemeinen, wie oben angegeben, 1 - 2 Mol Chlorcyan und 1 - 1,5 Moläquivalente einer Hilfsbase, vorzugsweise 1,01 - 1,5 Mol Chlorcyan und 1,0 - 1,3 Moläquivalente einer Hilfsbase, und besonders bevorzugt 1,05 - 1,3 Mol Chlorcyan und 1.0 - 1,15 Moläquivalente der Hilfsbase ein; hierbei soll zu jedem Zeitpunkt der Reaktion das Molverhältnis von Chlorcyan zur Hilfsbase größer als 1 sein.

Die Aufarbeitung und Isolierung der Reaktionsprodukte richtet sich nach dem jeweils eingesetzten Arylamin (I) und der damit verbundenen Reaktionsstöchiometrie.

Im einfachsten Fall, wenn das entstandene Cyanamid vollständig ausgefallen ist, kann es durch Absaugen isoliert werden. Muß dagegen bis Ende der Reaktion ein homogenes Medium aufrechterhalten werden, damit nicht Amin mitgefällt wird und dadurch der Umsetzung entgeht, so kann

entweder das Reaktionsgemisch in Wasser verrührt werden, oder es wird zuvor unter vermindertem Druck vorsichtig der größte Teil der Essigsäure abdestilliert und das stark eingeengte Reaktionsgemisch wird anschließend mit Wasser versetzt; hierdurch werden jeweils die Salze (Chloride) der Hilfsbase in Lösung gebracht oder gehalten und die gebildeten Cyanamide ausgefällt, welche dann wiederum durch Absaugen isoliert werden können.

Cyanamide extrem schwach-basischer, primärer Arylamine (welche manchmal nicht sofort sehr rein anfallen) können am zweckmäßigsten durch Umfällen und anschließendes Absaugen isoliert werden. Diese Methode besteht darin, daß das zunächst erhaltene Rohprodukt mit Alkalihydroxidlösung behandelt wird, daß geringe Mengen unlöslicher Anteile durch Abfiltrieren entfernt werden und daß das gewünschte Cyanamid anschließend durch vorsichtiges Ansäuern des Filtrats, welches die alkalilöslichen Anteile enthält, wieder ausgefällt wird.

Die erfindungsgemäß herstellbaren Aryl-cyanamide (II) sind Feststoffe. Ihre Charakterisierung durch Schmelzpunktbestimmung ist jedoch problematisch wegen der in den meisten Fällen eintretenden thermischen Zersetzung (vgl. "Analytik" im Beispielteil).

Die erfindungsgemäß herstellbaren Aryl-cyanamide können als Zwischenprodukte zur Herstellung z.B. von entzündungshemmenden Verbindungen (vgl. JP-A-55-141472), von Coccidiostatica (vgl. US-A-3.830.928), von Sedativa, Analgetica und Narkotica (vgl. BE-A 872.163) sowie von Diuretica (vgl. DE-A-2.251.354) verwendet werden. Ferner sind die

Bis- und Tris-Cyanamide der allgemeinen Formel (II), mit n=2 oder 3, wertvolle polymerisierbare Monomere, da aus ihnen polymere Verbindungen hergestellt werden können, welche z.T. hochentwickelte filmbildende Eigenschaften besitzen (vgl. DE-A 2.019.214).

Die nachfolgenden Beispiele dienen zur weiteren Erläuterung der Erfindung.

Beispiele

Einige der erfindungsgemäß hergestellten Cyanamide sind neu.

zur Analytik:

Reinheitskriterium der erhaltenen Cyanamide primärer Arylamine war - da fast alle Schmelzpunkte Zersetzungspunkte sind - die vollständige Löslichkeit in verdünnter wäßriger Natronlauge (10 % NaOH) sowie die Reinheit nach Dünnschichtchromatogramm (DC) durch Doppelbestimmung mit unterschiedlichen Laufmittelgemischen unter Verwendung der möglichen Nebenprodukte (d.h. Amine und Harnstoffe) als Vergleichssubstanzen.

Alle erfindungsgemäß hergestellten Aryl-cyanamide erwiesen sich als Reinsubstanzen; sie waren - sofern sie sich von primären Arylaminen ableiten - in verdünnter Natronlauge völlig löslich, und im DC waren keine Verunreinigungen feststellbar. Alle Cyanamide zeigen im IR-Spektrum eine intensive, breite, teilweise aufgespaltene CN-Bande (im Bereich : 2170 - 2230 $cm^{-1}$).

Die Aryl-cyanamide können auch massenspektrometrisch durch ihre Molekülionen charakterisiert werden (teilweise mittels GC/MS-Kopplung).

Beispiel 1

4-Chlorphenylcyanamid (erfindungsgemäß):

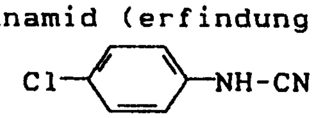

12,8 g (0,1 Mol) 4-Chloranilin wurden in 50 ml Eisessig gelöst und mit 350 ml Wasser versetzt: Bei 10°C wurden zu

dieser homogenen Lösung 6,1 ml (0,12 Mol) Chlorcyan gegeben und innerhalb 15 min 110 ml 1 N NaOH unter leichter Kühlung zugetropft. Es wurde noch 30 min nachgerührt, abgesaugt, gewaschen und getrocknet.

Man erhielt 12,7 g 4-Chlorphenylcyanamid (84 % d. Th.), welches nach dünnschichtchromatographischer Untersuchung keine Verunreinigung enthielt; die Substanz war in 2 N NaOH klar löslich.

<u>Vergleichsbeispiel 1a</u>

4-Chlorphenylcyanamid

analog: Synthesis <u>1976</u>, S. 592
(Bromcyan wurde durch Chlorcyan ersetzt, schwach-basisches Amin)

Zu einer Lösung, welche 5,1 ml (0,1 Mol) Chlorcyan in 100 ml Tetrahydrofuran enthielt, wurde nach Zugabe von 27,6 g (0,2 Mol) Kaliumcarbonat bei ca. -15°C eine Lösung von 12,7 g (0,1 Mol) 4-Chloranilin in 100 ml Tetrahydrofuran zugetropft. Nach weiteren 2 Stunden war im Dünnschichtchromatogramm keine Reaktion festzustellen. Erst beim Anwärmen auf Raumtemperatur setzte eine schwach exotherme Reaktion ein und dünnschichtchromatographisch ließ sich das Auftreten von 4-Chlorphenylcyanamid nachweisen. Nach beendeter Reaktion wurde eingeengt. Der Rückstand wurde mit Methylenchlorid und Natriumcarbonatlösung verrührt, die alkalisch-wäßrige Phase wurde abgetrennt und angesäuert. Es fielen 2,5 g (16,5 % d. Th.) 4-Chlorphenylcyanamid an.

Die analoge Durchführung mit BrCN lieferte 5,3 g (35 % d. Th.) 4-Chlorphenylcyanamid.

Vergleichsbeispiel 1b

4-Chlorphenylcyanamid

analog: M.P. Pierron, Bull. Soc. Chim. 35, S. 1203 (1906) (Chlorcyan und 4-Chloranilin anstelle von Bromcyan und 4-Bromanilin)

10 g (78,4 mMol) 4-Chloranilin, 200 ml Ethanol und eine Lösung aus 8,8 g (87,7 mMol) Kaliumhydrogencarbonat in 40 ml Wasser wurden mit 4,2 ml (82,8 mMol) Chlorcyan versetzt und 18 Stunden bei Raumtemperatur gerührt. Anschließend wurde mit 11,06 g (80 mMol) Kaliumcarbonat alkalisch gestellt und eingeengt. Der Rückstand wurde mit 200 ml Wasser versetzt (pH 9 - 10), dann wurde abgesaugt und die alkalische Lösung angesäuert. Es fielen 3,1 g (26 % d. Th.) (dünnschichtchromatographisch sauberes) 4-Chlorphenylcyanamid aus.

Vergleichsbeispiel 1c

4-Chlorphenylcyanamid

analog: Journal of the Indian Institute of Science, 29 A. S. 3 (1946); ≙ C. A. 41; 6214 h
(die Vorschrift ist der von Pierron ähnlich)

Anstelle von Bromcyan wurden 7,2 ml (0,1415 Mol) Chlorcyan eingesetzt. Es konnte 1 g (46 % d. Th.) 4-Chlorphenylcyanamid isoliert werden.

Beispiel 2

3,4-Dichlorphenylcyanamid (erfindungsgemäß)

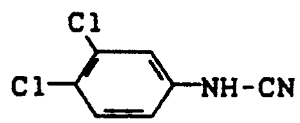

Variante a

163 g (1 Mol) 3,4-Dichloranilin wurden in 1985 g Essigsäure (90 %ig) gelöst und anschließend bei 10°C mit 61,2 ml (1,2 Mol) Chlorcyan versetzt. Anschließend wurden bei 5 - 10°C 1100 ml 1 N NaOH zulaufen gelassen, dann wurde 2 Stunden nachgerührt, abgesaugt, der Rückstand mit Wasser gewaschen und getrocknet.

Man erhielt 144,5 g 3,4-Dichlorphenylcyanamid. Durch Verdünnen der Mutterlauge mit Wasser fielen weitere 27,4 g Produkt aus. Beide Fraktionen waren in ca. 2 N NaOH klar löslich und nach dünnschichtchromatographischer Untersuchung einheitlich. Die Gesamtausbeute an reinem 3,4-Dichlorphenylcyanamid betrug somit 171,9 g (92 % d. Th.).

Variante b

16,2 g (0,1 Mol) 3,4-Dichloranilin wurden in 278 g Essigsäure (64 %ig) gelöst; bei 10°C wurden 6,1 ml (0,12 Mol) Chlorcyan zugegeben und sodann langsam 22 g (0,11 Mol) 20 %ige NaOH-Lösung unter Kühlung zugetropft. Es wurde 2 Stunden bei Raumtemperatur nachgerührt, abgesaugt, der Filterrückstand mit Wasser gewaschen und getrocknet. Man

erhielt 15 g (≙ 80 % d. Th.) an 3,4-Dichlorphenylcyanamid, welches klar in 2 N NaOH löslich und nach dünnschichtchromatographischer Untersuchung einheitlich war.

Beim Verdünnen der Mutterlauge mit Wasser fielen noch 2 g (≙ 11 % d. Th.) an sauberem 3,4-Dichlorphenylcyanamid aus. Die Gesamtausbeute lag somit bei 26 g (≙ 91 % d. Th.).

Obige Reaktion wurde wiederholt, jedoch mit 200 ml Essigsäure (90 %ig). Nach beendeter Reaktion wurde eingeengt und der Rückstand mit Wasser gewaschen. Ausbeute 16,8 g (≙ 90 % d. Th.), nach dünnschichtchromatographischer Untersuchung einheitliches Produkt.

Variante c

16,3 g (0,1 Mol) 3,4-Dichloranilin wurden in 200 ml Eisessig gelöst, bei 14°C mit 6,1 ml (0,12 Mol) Chlorcyan versetzt und unter Kühlung bei 10°C wurde langsam eine Lösung von 7,6 g (0,055 Mol) Kaliumcarbonat in 10 ml Wasser zugetropft. Es wurde 2 Stunden bei Raumtemperatur nachgerührt, dann im Wasserstrahlvakuum eingeengt, mit 10 %iger NaOH verrührt, abgesaugt und die Lösung angesäuert.

Man erhielt 15,1 g (≙ 81 % d. Th.) 3,4-Dichlorphenylcyanamid, welches nach dünnschichtchromatographischer Untersuchung einheitlich war.

Variante d

16,3 g (0,1 Mol) 3,4-Dichloranilin wurden in 278 g Essigsäure (64 %ig) gelöst und bei 10°C mit 6,1 ml (0,12

Mol) Chlorcyan versetzt; bei einer Temperatur unter 10°C wurden langsam insgesamt 9,2 g (0,11 Mol) Natriumhydrogencarbonat zugegeben.

Nach 2 Stunden wurde abgesaugt, der Filterrückstand mit Wasser gewaschen und getrocknet. Man erhielt 16,4 g (≙ 88 % d. Th.) 3,4-Dichlorphenylcyanamid. Die Substanz war in 2 N NaOH löslich und nach dünnschichtchromatographischer Untersuchung einheitlich.

Variante e

16,3 g (0,1 Mol) 3,4-Dichloranilin wurden in 150 ml Ethanol und 40 g Essigsäure (50 %ig) bei einer Temperatur unter 10°C mit 6,1 ml (0,12 Mol) Chlorcyan versetzt. Anschließend wurden 110 ml 1 N NaOH-Lösung zugetropft. Es wurde 1 Stunde nachgerührt, im Wasserstrahlvakuum bis 30°C Badtemperatur eingeengt und der Rückstand mit Wasser gewaschen. Man erhielt 17,8 g (≙ 95 % d. Th.) 3,4-Dichlorphenylcyanamid. Die Substanz war nach dünnschichtchromatographischer Untersuchung einheitlich und in 2 N NaOH klar löslich.

Vergleichsbeispiel 2a

analog: Tetrahedron 38, S. 1771 (1982)
(Bromcyan wurde durch Chlorcyan ersetzt, außerdem wurde ein schwach-basisches Amin eingesetzt)

163 g (1 Mol) 3,4-Dichloranilin und 61,2 ml (1,2 Mol) Chlorcyan wurden in 250 ml 1 M Essigsäure/Natriumacetat 4 Stunden bei Raumtemperatur gerührt. Anschließend wurde

abgesaugt, der Filterrückstand mit Wasser gewaschen und getrocknet. Man erhielt 171,5 g Rohprodukt. Hiervon wurden 81,1 g mit ca. 2 N NaOH digeriert und von unlöslichem Rückstand abfiltriert; das Filtrat wurde angesäuert, der ausgefallene Niederschlag wurde abfiltriert, mit Wasser gewaschen und getrocknet. Es verblieben 40,4 g 3,4-Dichlorphenylcyanamid, welches nach dünnschichtchromatographischer Untersuchung noch geringe Mengen von 2 unbekannten Verunreinigungen enthielt. Die Ausbeute nach Reinigung lag somit bei 45,4 % d. Th. Der alkaliunlösliche Anteil bestand nach dünnschichtchromatographischer Untersuchung zum größten Teil aus nicht umgesetztem 3,4-Dichloranilin.

Vergleichsbeispiel 2b

Analog zu obiger Vorschrift 2a wurden 0,2 Mol 3,4-Dichloranilin mit 0,4 Mol Chlorcyan umgesetzt. Nach Aufarbeitung über Natronlauge erhielt man 59 % d. Th. an gewünschtem Produkt, welches nach dünnschichtchromatographischer Untersuchung noch eine geringe Menge einer unbekannten Verunreinigung enthielt.

Beispiel 3

3-Nitrophenylcyanamid (erfindungsgemäß)

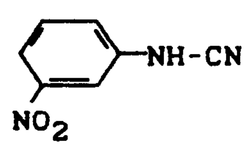

13,8 g (0,1 Mol) 3-Nitroanilin wurden in 190 g Essigsäure (79 %ig) gelöst und bei 10°C mit 6,1 ml (0,12 Mol)

Chlorcyan versetzt; innerhalb 3 Stunden wurden 110 ml 1 N NaOH zugetropft. Anschließend wurde eingeengt, mit ca. 2 N NaOH digeriert, filtriert, das Filtrat angesäuert, das ausgefallene Produkt abgesaugt, gewaschen und getrocknet.

Man erhielt 13,1 g (80,4 % d. Th.) 3-Nitrophenylcyanamid, welches nach dünnschichtchromatographischer Untersuchung einheitlich war.

Vergleichsbeispiel 3

3-Nitrophenylcyanamid

analog: Tetrahedron 38, S. 1771 (1982)
(Bromcyan wurde durch Chlorcyan ersetzt; schwach-basisches Amin)

13,8 g (0,1 Mol) 3-Nitroanilin und 25,5 ml (0,5 Mol) Chlorcyan wurden in 250 ml 1 M Essigsäure/Natriumacetat 4 Stunden bei Raumtemperatur gerührt. Anschließend wurde abgesaugt, der Niederschlag mit ca. 2 N NaOH digeriert und der alkalilösliche Teil durch Ansäuern wieder ausgefällt. Man erhielt 10,5 g (64 % d. Th.) 3-Nitrophenylcyanamid, welches nach dünnschichtchromatographischer Untersuchung noch eine geringe Menge einer Verunreinigung enthielt.

Beispiel 4

1,4-Biscyanaminobenzol (erfindungsgemäß)

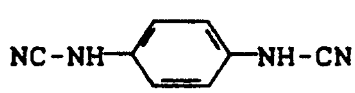

21,6 g (0,2 Mol) p-Phenylendiamin wurden in 500 ml 90 %iger wäßriger Essigsäure gelöst und bei 15°C mit 24,4 ml (0,48 Mol) Chlorcyan versetzt; dann wurden 440 ml 1 N NaOH zugetropft. Anschließend wurde abgesaugt, der Filterrückstand mit Wasser gewaschen und getrocknet. Man erhielt 28,6 g (90 % d. Th.) an 1,4-Biscyanaminobenzol. Die Substanz war nach dünnschichtchromatographischer Untersuchung einheitlich und in 2,5 N NaOH sowie verdünntem Ammoniak klar löslich.

Beispiel 5

4,4'-Biscyanaminodiphenylmethan (erfindungsgemäß)

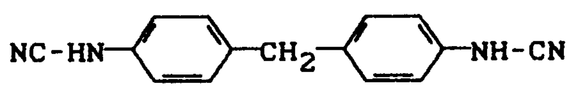

594 g (3 Mol) 4,4'-Diaminodiphenylmethan wurden in 3 l Eisessig und 300 ml Wasser gelöst, bei 10 - 15°C mit 366 ml (7,2 Mol) Chlorcyan versetzt; anschließend wurden langsam 2200 ml (6,6 Mol) 3 N NaOH zugesetzt.

Der ausgefallene Feststoff wurde abgesaugt und gewaschen; aus der Mutterlauge wurde durch Verdünnen eine weitere Fraktion gefällt. Insgesamt wurden nach Trocknen im Vakuumtrockenschrank 696 g (93 % d. Th.) an 4,4'-Biscyanaminodiphenylmethan erhalten. Die Substanz war nach dünnschichtchromatographischer Untersuchung einheitlich und in verdünnter Natronlauge klar löslich.

Analog zu Beispiel 5 kann auch die folgende Verbindung hergestellt werden (Beispiel 6).

Beispiel 6

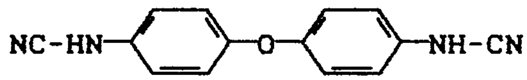

(Ausbeute: 84 % d.Th)

Analog zu den vorausgehenden Beispielen sind auch die in der nachfolgenden Tabelle 1 aufgeführten Verbindungen der Formel (II) hergestellt worden, wobei aber hinsichtlich maximaler Ausbeute nicht optimiert wurde:

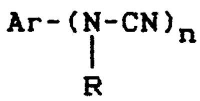 (II)

Tabelle 1

| Bsp. Nr. | Ar | R | n | Ausbeute |
|---|---|---|---|---|
| 7 | 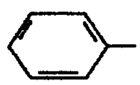 | H | 1 | *) |
| 8 | 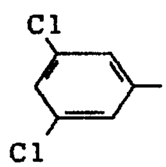 | H | 1 | 81 % |
| 9 | 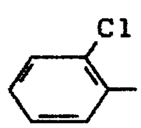 | H | 1 | 82 % |
| 10 | 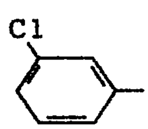 | H | 1 | 80 % |

*) IR-Spektrum: CN-Gruppe; Produkt ist alkali-löslich, zersetzt sich beim Trocknen über Nacht; deshalb ist keine exakte Ausbeuteangabe möglich.

Tabelle 1 (Fortsetzung)

| Bsp. Nr. | Ar | R | n | Ausbeute |
|---|---|---|---|---|
| 11 | 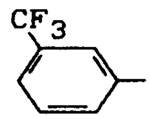 | H | 1 | 79 % |
| 12 | 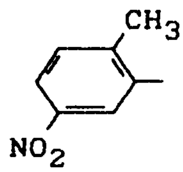 | H | 1 | 85 % |
| 13 | 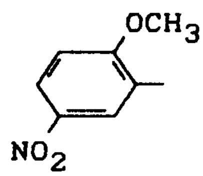 | H | 1 | 91 % |
| 14 | 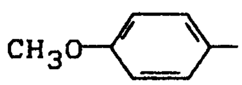 | H | 1 | 70 % |
| 15 | 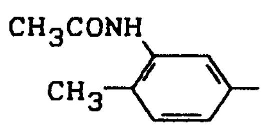 | H | 1 | 74 % |
| 16 | 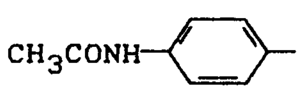 | H | 1 | 85 % |
| 17 | 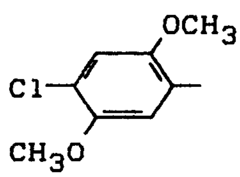 | H | 1 | 79 % |

Tabelle 1 (Fortsetzung)

| Bsp. Nr. | Ar | R | n | Ausbeute |
|---|---|---|---|---|
| 18 | 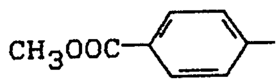 | H | 1 | 73 % |
| 19 | 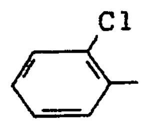 | $C_2H_5$ | 1 | 71 % |
| 20 | 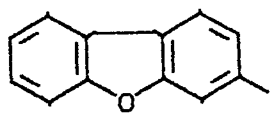 | H | 1 | 95 % |
| 21 | 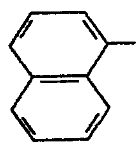 | H | 1 | 75 % |
| 22 | 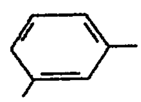 | H | 2 (1,3-Stellung) | 90 % |
| 23 | 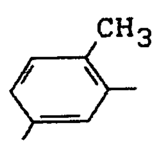 | H | 2 (1,3-Stellung) | 98,5 % |
| 24 | 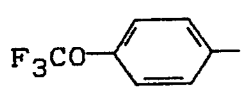 | H | 1 | 96 % |

## Patentansprüche

1. Verfahren zur Herstellung von Aryl-cyanamiden durch Umsetzung von Arylaminen der allgemeinen Formel I

$$Ar - \underset{\displaystyle R}{\underset{|}{(NH)_n}} \qquad (I)$$

worin

Ar für gegebenenfalls zusätzlich substituiertes Aryl steht,

R für Wasserstoff oder Alkyl steht und

n die Zahlen 1, 2 oder 3 bedeutet,

(wovon jedoch 2-Nitro- und 4-Nitroanilin sowie solche Arylamine, die eine gleich geringe oder eine noch geringere Nucleophilie als 2- bzw. 4-Nitroanilin besitzen, ausgenommen sind),

mit Chlorcyan (Cl-CN), dadurch gekennzeichnet, daß man die Reaktion zu Beginn in homogener flüssiger Phase durchführt unter Verwendung von Essigsäure (welche gegebenenfalls durch Wasser und/oder durch ein mit Wasser mischbares organisches Hilfslösungsmittel verdünnt ist) als Reaktionsmedium, und daß man auf jede Aminogruppe pro Mol Arylamin (I) 1 - 2 Mol Chlorcyan und 1 - 1,5 Moläquivalente einer

Hilfsbase einsetzt, wobei zu jedem Zeitpunkt der Reaktion dem Reaktionsgemisch mehr Chlorcyan als Hilfsbasenäquivalente zugesetzt werden, so daß der pH-Wert des Reaktionsgemisches unterhalb von 7 bleibt.

2. Verfahren nach Anspruch 1, dadurch gekennzeichnet, daß die Umsetzung bei Temperaturen von -20° C bis +60° C durchgeführt wird.

3. Verfahren nach Anspruch 2, dadurch gekennzeichnet, daß die Umsetzung bei Temperaturen zwischen 0° C und 40° C durchgeführt wird.

4. Verfahren nach Anspruch 3, dadurch gekennzeichnet, daß die Umsetzung bei Temperaturen zwischen 5 und 25° C durchgeführt wird.

5. Verfahren nach Anspruch 1, dadurch gekennzeichnet, daß man auf jede Aminogruppe pro Mol Arylamin (I) mindestens 1 Mol Essigsäure einsetzt.

6. Verfahren nach Anspruch 1, dadurch gekennzeichnet, daß man auf jede Aminogruppe pro Mol Arylamin (I) 1-2 Mol Chlorcyan und 1 - 1,5 Moläquivalente einer Hilfsbase einsetzt.

7. Verfahren nach Anspruch 6, dadurch gekennzeichnet, daß man auf jede Aminogruppe pro Mol Arylamin (I) 1,01 - 1,5 Mol Chlorcyan und 1,0 - 1,3 Moläquivalente einer Hilfsbase einsetzt.

8. Verfahren nach Anspruch 7, dadurch gekennzeichnet, daß man auf jede Aminogruppe pro Mol Arylamin (I) 1,05 - 1,3 Mol Chlorcyan und 1,0 - 1,15 Moläquivalente einer Hilfsbase einsetzt.

9. Verfahren nach Anspruch 1, dadurch gekennzeichnet, daß man als Hilfslösungsmittel Ethanol einsetzt.

10. Verfahren nach Anspruch 1, dadurch gekennzeichnet, daß man als Hilfsbase wäßrige Natriumhydroxid- oder Kaliumhydroxid-Lösung einsetzt.